# EUROPEAN PATENT SPECIFICATION

(11) **EP 0 501 950 B1**
(45) Date of publication and mention of the grant of the patent: **15.05.1996**
(21) Application number: 89912234.5
(22) Date of filing: 24.10.1989
(51) Int. Cl.: C12Q 1/68, G01N 33/543

(54) **METHOD FOR THE DIAGNOSING OF VIRAL AND GENETIC DISEASES**
VERFAHREN ZUR DIAGNOSTIZIERUNG VON VIRALEN UND GENETISCHEN KRANKHEITEN
PROCEDE DE DIAGNOSTIC DE MALADIES VIRALES ET GENETIQUES

(43) Date of publication of application: 09.09.1992
(73) Proprietor: ORGENICS LTD., Yavne 70 650 (IL)
(72) Inventor: NUR, Israel, 76 100 Rehovot (IL); HERZBERG, Max, Satarya (IL); ELKAIM, René, F-67000 Strasbourg (FR)
(74) Representative: Ramey, Daniel
(86) International application number: EP8901275
(87) International publication number: WO9106659

(56) References cited:
- EP-A- 0 211 777
- DIALOG INFORMATION SERVICES, File 154, Medline 83-90; B. DUTILH et al., AN 88251786/
- JOURNAL OF CANCER RESEARCH & CLINICAL ONCOLOGY, vVol. 108, 1984; P. NEHLS et al., pp. 23-29/

## Description

The present invention relates to a method and apparatus for diagnosis of at least one disease by assaying for the presence and/or quantity of a target suspect nucleic acid. More specifically it combines and improves the assay based on non-radioactive labeling of DNA or RNA probes used in their detection by the hybridization technique, and the solid phase immunoassay technique of the dipstick flat card having a series of longitudinal projections on which the known, non-labeled nucleic acid sequence complementary to the target suspect nucleic acid sequence are selectively positioned. The present invention is also useful for the simultaneous diagnosis of different diseases in a sample.

The present method and apparatus is particularly suitable for detection of target suspect nucleic acids and provides a simple, inexpensive, quantitative and reliable diagnosis of infectious and genetic diseases, in a plurality of individual samples collected from different patients.

There is an obvious diagnostic value in an apparatus for DNA probing that may be performed easily, rapidly, simply and with high accuracy by non-specialized personnel having limited laboratory skills and instrumentation. In addition, the present method and apparatus provides an adapted reaction container having a plurality of individual compartments and especially advantageous microprocessor-controlled reflectometer enabling accurate measuring of color density of the said assay.

This application is related to the subject matter of EP0 128 018, EP0 171 150 and FR 85 16533. In the above references there is extensive review of prior state of the art techniques related to the main elements of the present invention, which are as follows :
1. Nucleic acids in a partially purified or unpurified biological sample are specifically modified in the presence of sodium bisulfite at high molarity resulting in the addition of an antigenic sulfone group to C-6 of the cytosine moieties on the single-stranded DNA. The resulting sulfone group is stabilized by amination of cytosine bases with --o-methyl-hydroxylamine--, resulting in the substitution of an amine group on carbon 4.
2. The sample containing modified DNA or RNA is then mixed with hybridization solution and transferred to the first compartment in the reaction container. The plastic flat card having a group of projections each containing an unlabeled probe(s), is inserted into the above hybridization solution in the said compartment. Hybridization is carried out for 1 - 18 hours.
3. After the hybridization, the labeled target DNA or RNA is visualized for instance by immunochemical reactions carried out in subsequent compartments of the reaction container as follows : 1) Posthybridization wash, in the second compartment ; 2) Incubation with labeled antibody against sulfonated DNA in the next compartment ; 3) Wash compartment ; and 4) Incubation with chromogenic substrate to produce a color spot in the next compartment. The intensity of the color spot is proportional to the amount of specific DNA in the given sample. Quantitative results can be obtained by using the CombScan^{R} , a specially adapted microprocessor-controlled reflectometer which translates the intensity of tne colored spot into numerical units.

While the hybridization of DNA or RNA immobilized on solid supports is a known and fundamental technique in biochemistry and molecular biology for the detection of specific nucleic acid sequences, the commonly employed procedures, such as dot hybridization, Southern blots and colony and plaque hybridization utilize either filtration of a treated biological sample or immobilization of the sample followed by their processing. The above techniques utilized nitrocellulose or nylon membranes predominantly. However, the low retention efficiency and relative inaccessibility of the immobilized nucleic acid to the probe due to only a small part of the target nucleic acid available for hybridization, largely limits the use of this technique for diagnostic purposes. Therefore to overcome the above shortcomings and to combine at its best the two known assay systems, the present inventors have provided a reversed hybridization method and apparatus where an unlabeled known nucleic acid sequence is attached to solid support and utilized in a hybridization reaction with a chemically modified target sample DNA or RNA. Visualization is achieved by immunological reactions, especially adapted to achieve the highest sensitivity in the shortest period of time.

The present invention enables the detection of 10 pg of homologous DNA or RNA in 1 - 2 hours and the detection of 1 pg in overnight hybridization, with all required chemicals provided in the kit.

Although the invention has been described with reference to particular means, materials and procedures it is to be understood that the invention is not limited to the particulars disclosed and extends to all equivalents falling within the scope of the claims.

The present invention is particularly useful in detecting the presence and/or quantity of target nucleic acids such as DNA or RNA having at least 50 bases. The target nucleic acid preferably may be partially extracted from any biological sample suspected of being contaminated.

Various known techniques of amplification of target nucleic acid may be used. Biological sample containing at least 50 pg of total target DNA per sample may be used without amplification. Alternatively, for lower concentrations PCR amplification technique described (in Trends in Genetics, June 1989 Vol. 5 No. 6) can be used for amplification of the specific DNA to above the desired concentrations.

### EXAMPLE 1

### Labeling of target nucleic acid sequence and transfer to hybridization container's compartment.

The cells were treated, their DNA partially extracted and completely sulfonated as follows :

Biopsies or cell cultures were incubated for 30 minutes at 55°C in lysis buffer (0.05 M Tris-HCl pH = 8.0, 0.05 M NaEDTA, 0.5 % SDS and 100 ug/mL Proteinase). This lysate is then diluted by adding four volumes of water, mixed and boiled for 10 minutes. The partially purified partially denatured lysed sample was cooled on ice and the modification solutions were added as described in the Chemiprobe^{R} instruction manual. The labeling is accomplished by inserting an antigenic sulfone group into cytosine moieties of ssDNA (1). The cytosines are sulfonated at carbon 6 by sodium bisulfite at high molarity (modification solution A). The resulting sulfone is stabilized by amination with --o-methyl-hydroxylamine (modification solution B) resulting in the substitution of the amine group on carbon 4. In this way, cytosines are transformed into N-4-methoxy-5, 6-dihydro-cytosine-6-sulfonate. Based upon HPLC studies, it is estimated that about 10 - 15 % of the cytosine residues in the DNA molecule are sulfonated. This is equivalent to 2 - 3 % of all nucleotide residues in a DNA with 47 % G - C content.

After overnight sulfonation 0.6 volume of propanol were added to the modification solutions in order to desalt the modified DNA. This mixture was then centrifuged for 10 minutes in a microcentrifuge at 10,000xg. The supernatant was then decanted and the pellet washed with 70 % ethanol, air dried and suspended in 200 uL of hybridization solution in order to be transferred into the proper hybridization container.

### EXAMPLE 2

### Loading of DNA on activated tabs of solid planar support card.

The following plasmids : pBR322 and HBV, HPV DNA sequences cloned in pUC were used. Each plasmid, 40 ng per 4 µL drop, was linearized by sonicating for 20 seconds per mL with 50 % intervals, using the microtip at position 5 of the output control, output of 120 watts. (Sonicator was Ultrasonic, Inc., W-385, Farmingdale, N.Y., USA). Alternatively, the plasmid was linearized by cleaving with the restriction enzyme EcoR I using 3 units of enzyme per µg of plasmid DNA for 3 hours.

The plasmid DNA was boiled and cooled on ice, and 4 µL droplets ds DNAs were placed on the tabs of high impact polystyrene or nitrocellulose. The droplets were dried by evaporation either by placing the card directly in an 80°C oven for 30 minutes or by first drying the sample in air for 1 hour.

The tabs were then blocked/coated in a solution of 2 M guanidine thiocyanate, 2 x SSC (0.3 M NaCl - 0.075 M sodium citrate), 40 mM EDTA, 1 % sarcosine, and 1.5 % poly(acrylic acid) ; M.W. 90,000 (Aldrich Chemical Co., Milwaukee, WI), for 15 minutes and then air dried for 15 min.

We found that using freshly made 1 M ammonium acetate is the best loading buffer as compared to other buffers (PBS, ammonium carbonate, Tris-HCl or ammonium citrate).

### EXAMPLE 3

### Hybridization

Hybridization reaction with known nucleic acid sequence positioned on the activated tabs, complementary to target DNA or RNA, is done as follows in the reaction container's compartment. Hybridization was carried out for 1 to 16 hours (overnight incubation) in the first compartment of the reaction container either in Solution A, containing 50 % deionized formamide, 0.7 M NaCl, 50 mM Tris-HCl, 1 % SDS and 1.5 % neutralized poly(acrylic acid) ; or in Solution B, containing 2 M guanidine thiocyanate. 2 x SSC (0.3 M NaCl + 0.075 M sodium citrate), 0.04 M NaEDTA 1 % sarcosine, and 1.5 % poly(acrylic acid). Both of the solutions were supplemented with 100 µg sonicated salmon sperm ssDNA.

Solution B gives lower background as compared to solution A and to other conventional hybridization solution. Solution B was used throughout the entire hybridization assays carried out in examples 3 to 6.

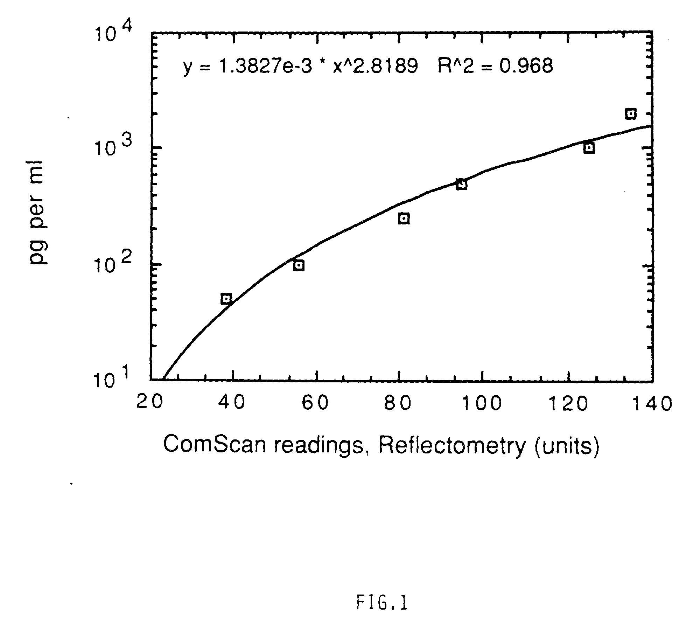

In an overnight hybridization one can detect clearly one pg of homologous DNA (Fig. 1). A simple calculation which takes into account the size of the human DNA and the number of bases participating in the homology will bring us to the conclusion that we will need 1 - 4 Kb of high homology in 1 µg of Human DNA in order to get a measurable signal.

### EXAMPLE 4

### Signal Generating System

Detection of chemically modified DNA or RNA may be carried out by a sandwich immunoenzymatic reaction. A monoclonal antiboty specifically recognizes the sulfone groups on the modified DNA. An enzyme-anti-immunoglobulin antibody conjugate then binds to the monoclonal antibody. The enzyme converts a soluble chromogenic substrate system (NBT-BCIP) into an insoluble dye which precipitates at the exact location of the immune reaction. The colored product indicates the presence of sulfonated DNA.

There are several alternatives for the above described visualization and one may prefer using fluorescein or colloidal gold labeled anti-mouse immunoglobulin, thereby circumventing interference from tissue enzymes. Other possibilities are to replace the alkaline phosphatase labeled conjugate with a conjugate of peroxidase or glucosidase. Optimization of the entire procedure (including washes and blocking solutions) should be performed before using these alternatives.

A biotinylated monoclonal against sulfonated DNA and streptavidine conjugated to alkaline phosphatase was successfully used giving much stronger reaction than the goat anti-mouse IgG conjugated to Alkaline Phosphatase.

Alternative Chromogens. The chromogenic system supplied may be 5 -bromo-4-chloro-3-indolyl phosphate (BCIP) and Nitro Blue Tetrasolium (NBT). BCIP can be used alone as chromogen with reduced sensitivity. A combination of Naphthol As-Mx phosphate and Fast Red yelds good sensitivity and red color, but is not as strong as BCIP-NBT.

### EXAMPLE 5

### Measuring and quantifying the reactions

CombScan^{R} is commercially available from Orgenics Ltd. and is a fast 8-bit microprocessor-controlled reflectometer. It takes about 60 readings of each tab and assigns a value between O - 255 to the amount of light that reaches the diode. Using ROM-etched algorith, the color spot is distinguished from the background and its value is stored. All readings are analyzed by comparison with the control, ratio calculation and translation of color densities to actual titers according to selectable programs.

### EXAMPLE 6

### Detection and Typing Human Papilloma Viruses (HPV)

Squamous cell carcinoma of the uterine cervix is one of the most common cancers among women. Recent studies using the technique of DNA hybridization and molecular cloning have provided the evidence for the presence of DNA sequences of HPV types 16 and 18 in the majority of cervical tumor cell lines in contrast, HPV types 6B and 11 are commonly found in biopsies of benign condilomas. The cervical carcinoma cell lines, HeLa and CaSki, were originated from a tumor biopsy. It is therefore appropriate that these cell lines be used as a model for designing and testing protocols for HPV.

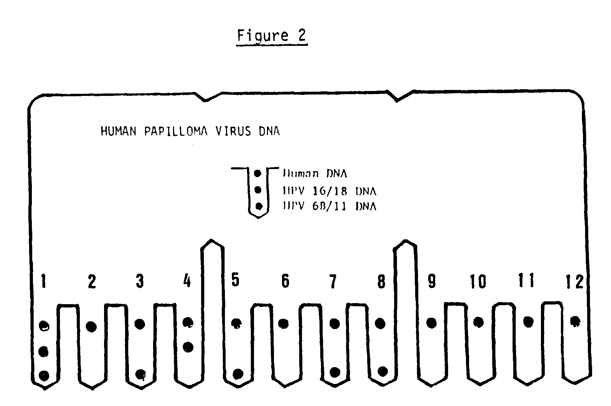

DNA was extracted from cervical biopsies and from CaSki and HeLa cells. (HeLa-ATCC CCL2 Epitheloid Carcinoma, cervix, Human, CaSki-ATCC CRL 1550 Human cervical epidermoid carcinoma. Cell lines were maintained in Dulbecco Modified Eagles medium supplemented with 100 % fetal calf serum, 2 mM glutamine, 100 µ/mL penicillin 100 µg/mL streptomycin and 0.25 µg/mL fungizone). The DAN was labeled and hybridized to three different probes immobilized on the solid support plastic card's tabs as described in Examples 1 to 3. Non-specific DNA human sequences were attached to middle spot and a probe with HPV 6 and 11 were bound to the lower spot as shown in Figure 2. The minimal number of CaSki and HeLa cells required to detect specific HPV sequences were respectively, 5 x 10³ and 5 x 10⁴ cells. In contrast, 10⁴ human embryologic cells gave no specific reaction. Eleven clinical biopsies were tested. Four were found to contain HPV 6/11 and one HPV 16/18. In the others we obtained a reaction only with the human non-specific sequences (figure 2).

5 x 10⁴ HeLa cells is equivalent to 1.5 µg of total human DNA as measured during preparation of genomic DNA integrated into the genome. The HPV homology region is about 1 Kb. The HeLa cell line we were using got only 2 - 4 copies of HPV 18 (Pater MM, Pater A (1985) Virology 145 : 313 - 318). Thus, we end up with sensitivity of about 1 pg of specific DNA. Using human papillomavirus types 16 and 18 sequences in carcinoma cell lines of the cervix, one needs about 5 x 10⁵ cells in order to detect a single gene of 1 Kb in the Hybricomb system. These results were confirmed also with CaSki cells (with 50 - 200 copies of HPV 16).

Figure 1. Correlation between the concentration of labeled DNA and the resulting color intensity obtained from DNA-DNA hybridization, 40 ng of pBR322 was immobilized on each tab and then hybridized for 16 hours to various concentrations of sulfonated pBR322 DNA. The intensity of the color precipitate was monitored by CombScan^{R}. The apparatus can detect 10 pg or nomologous DNA in 2 hours hybridization and 1 pg in an overnight hybridization assay.

Figure 2. Detection and typing of Human Papilloma Viruses (HPV) in cell cultures and patient biopsies. DNA was extracted from cervical biopsies and from CaSki and HeLa cells. The DNA was labeled and hybridized to three different immobilized probes on the plastic tab. A) A probe of non-specific DNA human sequences was immobilized on the upper spot B) A probe of HPV types 16 and 18 was attached to the middle spot and a probe for HPV types 6B and 11 was bound to the lower spot. The following specimens and controls were tested :

Tab 1 positive control ; No. 2) negative control (Human DNA), No. 3) human DNA with 2 Kb fragment of HPV 11. 4) 5 x 10⁴ CaSki cells. 5 to 12) Human biopsies.

### EXAMPLE 7

### Mycoplasma Detection and Typing

Cell cultures are often infected with mycoplasma. Since signs of mycoplasma contamination are typically unpronounced, "clean" cultures are not readily distinguishable from contaminated cultures. Among the mycoplasmal species infecting cell cultures, M. hyorhinis is often undetected since it frequently fails to grow in culture media. Thus, conventional detection procedures have typically relied on indirect methods such as DNA staining, enzymatic reactions, specific antibodies, and infection of indicator cell lines. All of these methods suffer from a degree of nonspecificity, low sensitivity, and high cost.

The present method overcomes these obstacles. 40 ng of total DNA extracted from six mycoplasmas species was linked to two separated tabs. Low speed centrifugation was used to separate the mycoplasmas and the cell culture. The mycoplasma cells from the low speed supernatant were pelleted by centrifugation in microcentrifuge for 10 minutes. The mycoplasma cells were boiled for 5 minutes for DNA extraction and sulfonation was done as described in Example 1 and carried out overnight. The labeled DNA was desalted by precipitation with an equal volume of propanol and the resulting pellet was dissolved in the hybridization buffer. Hybridization was carried out as described in Example 2.

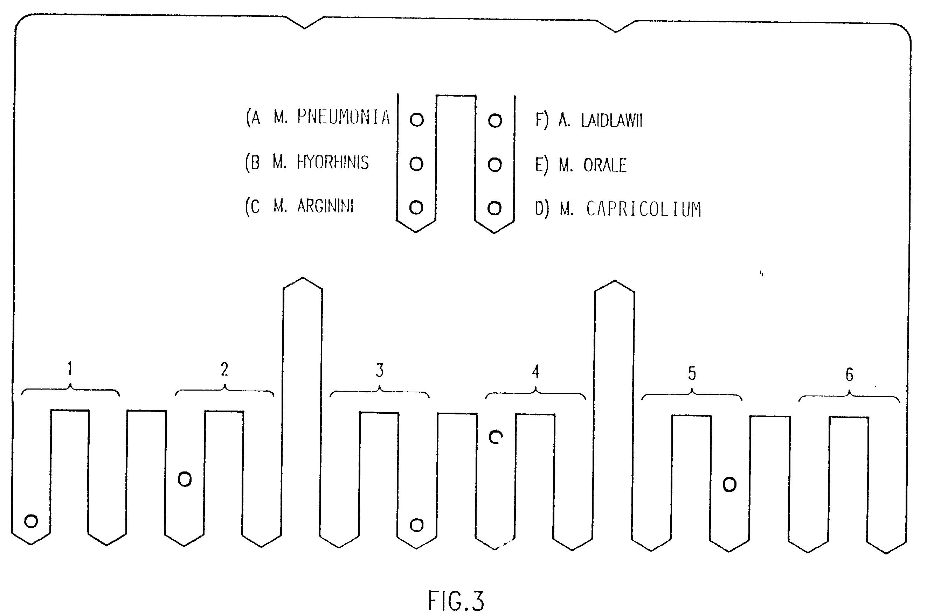

As can be seen in Figure 3 mycoplasmas can be detected and typed with high sensitivity and high specificity either in cell culture or in axcenic mycoplasma culture.

Figure 3. Detection and typing of mycoplasma in tissue culture. Mycoplasmal DNA from either axcenic mycoplasma cultures or from contaminated cell cultures were extracted and modified. Hybridization was carried out in the reaction container for 16 hours. Six different Mycoplasmal DNAs were spotted on six sets of two tabs each. Spotting was done counterclockwise on the two tabs beginning from the uppermost spot on the left tab to the upper spot on the right tab : a) M. pneumoniae ; b) M. hyorhinis ; c) M. arginini ; d) M. capricolium ; e) M. oral ; and f) Acholeplasma laidlawii. The tab pairs were then hybridized to the DNA extracted from the following mycoplasma : 1) M. arginini cells ; 2) M. hyorhinis cells ; 3 and 6 contamined tissue culture ; 4) 110 ng of M. pneumoniae total DNA ; and 5) M. orale cells.

Mycoplasma strains were grown as described by Leach. R.H. (1983). Preservation of mycoplasma cultures and culture collection. Methods in Mycoplasmology, Vo. I (eds. Razin and J.G. Tully), Academic Press, NY, pp. 197 - 204.

## Claims

1. A method for diagnosis of disease or the presence of a gene by assaying the presence and/or quantity of at least one target nucleic acid sequence in at least one partially denatured suspect sample, comprising the steps of :
a) modifying said partially denatured target nucleic acid sequence by sulfonation using compounds selected from the group consisting of bisulfites, and stabilizing the resulting sulfone by reaction with o-methyl-hydroxylamine.
b) contacting said modified target nucleic acid sequence of (a) with at least one known nucleic acid sequence complementary to said target nucleic acid sequences selectively positioned on projections of a solid planar support, to form an hybridized complex ;
c) contacting said hybridized complex (b) with an antibody specific to the said sulfonated portion of said target nucleic acid sequence, to form an antibodybound complex ;
Wherein said contacting (b) is made under hybridization conditions in a reaction container having a series of individual compartments for enabling said hybridized complex (b) to occur and additional individual compartments for carrying out separately, signal generating reactions by syccessive dipping of said projections.
d) measuring for the presence and/or quantity of said target nucleic acid sequence as an indication of said disease.

2. A method as defined by claim 1 wherein said solid planar support is a card with a series of projections on the edge of said card.

3. A method as defined by claim 2 wherein each of said projections bears one or more complementary nucleic acid sequences capable of forming a hybridized complex with said target nucleic acid sequences.

4. The method as defined by claim 2 further comprising in (b) at least one non-complementary acid sequence on the projections for the purpose of internal control of said assay.

5. The method as defined by claim 2 wherein said solid support is formed by high-impact polystyrene.

6. The method as defined by claim 1 wherein said measuring is made by computer means, reading the results of each of the reactions occurring on said solid support.

7. The method as defined by claim 1 wherein said antibody is a monoclonal antibody.

8. The method as defined by claim 1 wherein said antibody is labeled with an agent adapted to signal the presence of said antibody.

9. The method as defined by claim 1 wherein said agent is a labeled antibody.

10. A kit containing the means for carrying out the method of claim 1.

11. The method of claim 1 wherein said hybridization conditions includes the use of 2 M guanidine thiocyanate.

## Patentansprüche

1. Verfahren zur Diagnose einer Erkrankung oder des Vorhandenseins eines Gens durch Testen auf das Vorhandensein und/oder die Menge mindestens einer Zielnukleinsäuresequenz in mindestens einer teilweise denaturierten, verdächtigen Probe, dadurch **gekennzeichnet**, daß man in Stufen
a) die teilweise denaturierte Zielnukleinsäuresequenz durch Sulfonierung unter Verwendung von Verbindungen, ausgewählt aus der Gruppe bestehend aus Bisulfiten, modifiziert und das so erhaltene Sulfon durch Umsetzung mit 0-Methylhydroxylamin stabilisiert;
b) die modifizierte Zielnukleinsäuresequenz von (a) mit mindestens einer bekannten Nukleinsäuresequenz, die den Zielnukleinsäuresequenzen komplementär ist und selektiv auf Vorsprüngen eines festen Trägers angebracht sind, in Kontakt bringt, um einen hybridisierten Komplex zu bilden;
c) den hybridisierten Komplex (b) mit einem für den sulfonierten Anteil der Zielnukleinsäuresequenz spezifischen Antikörper in Kontakt bringt, um einen antikörpergebundenen Komplex zu bilden,
wobei man das in Kontakt bringen (b) unter hybridisierenden Bedingungen in einem Reaktionsgefäß mit einer Reihe von einzelnen Kompartmenten, die das Eintreten des hybridisierten Komplexes (b) erlauben, und zusätzlichen einzelnen Kompartmenten zur separaten Durchführung von signalerzeugenden Reaktionen durch aufeinanderfolgendes Eintauchen der Vorsprünge durchführt;
d) die Anwesenheit und/oder die Menge der Zielnukleinsäuresequenz als Indikation der Erkrankung mißt.

2. Verfahren nach Anspruch 1, wobei der feste planare Träger eine Karte mit einer Reihe von Vorsprüngen auf der Kante der Karte ist.

3. Verfahren nach Anspruch 2, wobei jeder der Vorsprünge eine oder mehrere komplementäre Nukleinsäuresequenzen trägt, die einen hybridisierten Komplex mit den Zielnukleinsäuresequenzen bilden können.

4. Verfahren nach Anspruch 2, umfassend weiter in (b) mindestens eine nichtkomplementäre Säuresequenz auf den Vorsprüngen zur internen Kontrolle des Assays.

5. Verfahren nach Anspruch 2, wobei der feste Träger durch ein Polystyrol mit hoher Schlagfestigkeit gebildet ist.

6. Verfahren nach Anspruch 1, wobei die Messung mittels Computer durchgeführt wird, wobei die Ergebnisse jeder der Reaktionen, die auf dem festen Träger stattfinden, abgelesen werden.

7. Verfahren nach Anspruch 1, wobei der Antikörper ein monoclonaler Antikörper ist.

8. Verfahren nach Anspruch 1, wobei der Antikörper mit einem Mittel markiert ist, das so angepaßt ist, daß es die Anwesenheit des Antikörpers anzeigt.

9. Verfahren nach Anspruch 1, wobei das Mittel ein markierter Antikörper ist.

10. Kit, umfassend die Mittel zur Durchführung des Verfahrens nach Anspruch 1.

11. Verfahren nach Anspruch 1, wobei die Hybridisierungsbedingungen die Verwendung von 2 M Guanidinthiocyanat umfassen.

## Revendications

1. Procédé pour le diagnostic d'une maladie ou la détection de la présence d'un gène, par détermination de la présence et/ou de la quantité d'au moins une séquence cible d'acide nucléique dans au moins un échantillon suspect partiellement dénaturé, comprenant les étapes de:
(a) modification de cette séquence cible d'acide nucléique partiellement dénaturée par sulfonation avec des composés choisis dans le groupe consistant en bisulfites, et stabilisation de la sulfone résultante par réaction avec de l'o-méthyl-hydroxylamine;
(b) mise en contact de cette séquence cible d'acide nucléique modifiée provenant de (a) avec au moins une séquence d'acide nucléique connue complémentaire de ces séquences cible d'acide nucléique disposée de façon sélective sur des prolongements d'un support plan solide, pour former un complexe hybridé;
(c) mise en contact de ce complexe hybridé (b) avec un anticorps spécifique de cette portion sulfonée de cette séquence cible d'acide nucléique, de façon à former un complexe lié à l'anticorps;
dans lequel cette mise en contact (b) est faite en conditions d'hybridation dans un récipient de réaction ayant une série de compartiments individuels pour permettre l'apparition de ce complexe hybridé (b) et des compartiments individuels supplémentaires pour réaliser, séparément, des réactions générant un signal par immersion successive de ces prolongements;
(d) mesure de la présence et/ou de la quantité de cette séquence cible d'acide nucléique comme indication de cette maladie.

2. Procédé suivant la revendication 1, dans lequel ce support plan solide est une carte avec une série de prolongements sur le bord de cette carte.

3. Procédé suivant la revendication 2, dans lequel chacun de ces prolongements porte une ou plusieurs séquences d'acide nucléique complémentaires capables de former un complexe hybridé avec ces séquences cibles d'acide nucléique.

4. Procédé suivant la revendication 2, comprenant de plus dans (b), au moins une séquence d'acide non complémentaire sur les prolongements afin de fournir un témoin interne de cet essai.

5. Procédé suivant la revendication 2, dans lequel ce support solide est formé par un polystyrène de haute résistance aux chocs.

6. Procédé suivant la revendication 1, dans lequel cette mesure est faite au moyen d'un ordinateur pour lire les résultats de chacune des réactions apparaissant sur ce support solide.

7. Procédé suivant la revendication 1, dans lequel cet anticorps est un anticorps monoclonal.

8. Procédé suivant la revendication 1, dans lequel cet anticorps est marqué avec un agent adapté pour signaler la présence de cet anticorps.

9. Procédé suivant la revendication 1, dans lequel cet agent est un anticorps marqué.

10. Kit contenant les moyens pour réaliser le procédé suivant la revendication 1.

11. Procédé suivant la revendication 1, dans lequel ces conditions d'hybridation comprennent l'utilisation de thiocyanate de guanidine 2 M.
